# EUROPEAN PATENT APPLICATION

(11) **EP 4 644 540 A1**
(43) Date of publication of application: **05.11.2025**
(21) Application number: 23910308.8
(22) Date of filing: 21.12.2023
(51) Int. Cl.: C12N 9/10, C12N 9/88, C12N 1/19, C12N 15/54, C12N 15/60, C12N 15/81, C12P 19/44, C12P 7/22, C12R 1/865

(54) **ENZYME, STRAIN FOR PRODUCING SALIDROSIDE, AND PRODUCTION METHOD**

(30) Priority: 29.12.2022 CN 202211708333
(71) Applicant: Bloomage Biotechnology Corporation Limited, Jinan, Shandong 250101 (CN)
(72) Inventor: LIU, Tengfei, Jinan, Shandong 250101 (CN); WANG, Huiyan, Jinan, Shandong 250101 (CN); ZHANG, Xiaofen, Jinan, Shandong 250101 (CN); CHEN, Tongtong, Jinan, Shandong 250101 (CN); WANG, Ruiyan, Jinan, Shandong 250101 (CN)
(74) Representative: Abel & Imray LLP
(86) International application number: PCT/CN2023/140455
(87) International publication number: WO 2024/140379

(57) **Abstract**

Provided is an application of glucosyltransferase U8GT3 and/or poppy-derived tyrosine decarboxylase or a mutant thereof in the yeast synthesis of salidroside; further provided is an application of poppy-derived tyrosine decarboxylase or a mutant thereof in the yeast synthesis of tyrosol. The amino acid sequences of the poppy-derived tyrosine decarboxylase and the mutant thereof are respectively shown in SEQ ID NOs: 7-8, and the nucleotide sequences of the coding genes are respectively shown in SEQ ID NOs: 1-2; the amino acid sequence of the glucosyltransferase U8GT3 is shown in SEQ ID NO: 10, and the nucleotide sequence of the coding gene is shown in SEQ ID NO: 4.

## Description

### TECHNICAL FIELD

The present application relates to the field of biotechnology, and in particular to an enzyme, a strain for producing salidroside and a production method, specifically to the use of glucosyltransferase U8GT3 and/or poppy-derived tyrosine decarboxylase and/or tyrosine decarboxylase mutants in improving the yield of salidroside synthesized by using yeast, the use of poppy-derived tyrosine decarboxylase and/or tyrosine decarboxylase mutants in improving the yield of tyrosol synthesized by using yeast, a strain for producing salidroside, a biological material, a whole-cell catalyst, and a method for producing salidroside and/or tyrosol by using yeast.

### BACKGROUND

Salidroside is an important active substance in the *Rhodiola rosea* plant. It has multiple physiological activities such as improving immunity, anti-aging, anti-radiation, anti-fatigue, and anti-Alzheimer's disease. It is widely used in functional cosmetics such as whitening, anti-aging, and anti-ultraviolet radiation.
At present, the salidroside on the market mainly relies on extraction from the rare wild plant *Rhodiola rosea* in high-altitude and pollution-free areas, but its content in the plant is only 0.5% to 0.8%. The extraction process is relatively complicated and is also restricted by seasonal climate and has high time and labor costs. The current chemical synthesis method is difficult to achieve industrialization due to the complicated preparation process. Faced with increasing market demand, the production of salidroside by using microbial cell factories has become a more promising option. The currently reported and publicized studies on the de novo synthesis of salidroside by yeast using glucose as a carbon source all involve the conversion of 4-hydroxyphenylpyruvate (4-HPP) into 4-hydroxyphenylacetaldehyde (4-HPAA) via phenylpyruvate decarboxylase (ARO10), followed by the Ehrlich pathway to synthesize the key precursor tyrosol. Although the metabolic flux of the target product can be improved through a series of metabolic pathway optimizations, the metabolic flux of its precursor still needs to be further improved for industrial production.

Therefore, designing and developing new precursor synthesis pathways and decoupling the cell growth of engineered yeast and the product synthesis of salidroside will greatly promote the industrial application of biosynthesis of salidroside and improve the economic benefits of fermentation production of salidroside.

### SUMMARY

In view of the deficiencies in the prior art, the present application provides an enzyme, a strain for producing salidroside and a production method, and specifically relates to the use of glucosyltransferase U8GT3 and/or poppy-derived tyrosine decarboxylase and/or tyrosine decarboxylase mutants in improving the yield of salidroside synthesized by using yeast, the use of poppy-derived tyrosine decarboxylase and/or tyrosine decarboxylase mutants in improving the yield of tyrosol synthesized by using yeast, a strain for producing salidroside, a biological material, a whole-cell catalyst, and a method for producing salidroside and/or tyrosol by using yeast.

The purpose of the present application is to overcome the existing technical difficulties. First, by knocking out the GAL80 gene in *Saccharomyces cerevisiae* and combining it with a galactose-inducible promoter, the cell growth of brewing engineered bacteria and the product synthesis of salidroside are decoupled, thereby reducing the metabolic pressure caused by the target pathway on the engineered bacteria, thereby achieving efficient and rapid production of salidroside. Secondly, the codon encoding amino acid at position 350 of the poppy (*Papaver somniferum*)-derived tyrosine decarboxylase (TYDC) gene is mutated to a codon encoding phenylalanine (TYDC^{Y350F}). The TYDC^{Y350F} mutant can directly catalyze the synthesis of 4-hydroxyphenylacetaldehyde (4-HPAA) from tyrosine, thereby constructing a new precursor synthesis pathway. Combining the aforementioned new and old metabolic pathways, the metabolic flow can be further directed to the synthesis of the target product salidroside, thereby improving the economic benefits of the fermentation production of salidroside.

Specifically, the present application involves the following aspects:
1. Use of glucosyltransferase U8GT3 and/or a poppy-derived tyrosine decarboxylase and/or a tyrosine decarboxylase mutant in improving the yield of salidroside synthesized by using yeast.
2. Use of a poppy-derived tyrosine decarboxylase and/or a tyrosine decarboxylase mutant in improving the yield of tyrosol synthesized by using yeast.
3. The use according to item 1 or 2, wherein
   the tyrosine decarboxylase mutant comprises an amino acid sequence represented by SEQ ID NO.7;
   the poppy-derived tyrosine decarboxylase comprises an amino acid sequence represented by SEQ ID NO.8; and
   the glucosyltransferase U8GT3 comprises an amino acid sequence represented by SEQ ID NO.10.
4. The use according to item 1, 2 or 3, wherein
   the tyrosine decarboxylase mutant comprises a nucleic acid molecule represented by SEQ ID NO.1;
   the poppy-derived tyrosine decarboxylase comprises a nucleic acid molecule represented by SEQ ID NO.2; and
   the glucosyltransferase U8GT3 comprises a nucleic acid molecule represented by SEQ ID NO.4.
5. A strain for producing salidroside, wherein the strain comprises a gene encoding glucosyltransferase U8GT3 and a gene encoding tyrosine decarboxylase,
   preferably, the gene encoding tyrosine decarboxylase is a gene encoding a poppy-derived tyrosine decarboxylase or a gene encoding a poppy-derived tyrosine decarboxylase mutant;
   more preferably,
   the glucosyltransferase U8GT3 comprises an amino acid sequence represented by SEQ ID NO.10;
   the poppy-derived tyrosine decarboxylase comprises an amino acid sequence represented by SEQ ID NO.8; and
   the poppy-derived tyrosine decarboxylase mutant is a tyrosine decarboxylase in which the tyrosine at position 350 is mutated to phenylalanine, and comprises an amino acid sequence represented by SEQ ID NO.7,
   most preferably, the yeast is *Saccharomyces cerevisiae.*
6. The strain according to item 5, wherein
   the poppy-derived tyrosine decarboxylase comprises a nucleic acid molecule represented by SEQ ID NO.2;
   the tyrosine decarboxylase mutant comprises a nucleic acid molecule represented by SEQ ID NO.1; and
   the glucosyltransferase U8GT3 comprises a nucleic acid molecule represented by SEQ ID NO.4.
7. The strain according to item 5 or 6, wherein,
   further, a gene encoding phenylpyruvate decarboxylase ARO10 and/or a gene encoding benzoate dehydrogenase TYR1 is heterologously expressed in yeast;
   preferably,
   the phenylpyruvate decarboxylase ARO10 comprises an amino acid sequence represented by SEQ ID NO.11; and
   the benzoate dehydrogenase TYR1 comprises an amino acid sequence represented by SEQ ID NO.12;
   more preferably,
   the phenylpyruvate decarboxylase ARO10 comprises a nucleic acid molecule represented by SEQ ID NO.5; and
   the benzoate dehydrogenase TYR1 comprises a nucleic acid molecule represented by SEQ ID NO.6.
8. A biomaterial, wherein the biomaterial is any one of the following:
   A1) a nucleic acid molecule encoding a poppy-derived tyrosine decarboxylase and/or a nucleic acid molecule encoding a tyrosine decarboxylase mutant;
   A2) an expression cassette comprising the nucleic acid molecule described in A1); and
   A3) a recombinant vector comprising the nucleic acid molecule described in A1), or a recombinant vector comprising the expression cassette described in A2);
   preferably,
   the tyrosine decarboxylase mutant comprises an amino acid sequence represented by SEQ ID NO.7; and
   the poppy-derived tyrosine decarboxylase comprises an amino acid sequence represented by SEQ ID NO.8;
   more preferably,
   the tyrosine decarboxylase mutant comprises a nucleic acid molecule represented by SEQ ID NO.1; and
   the poppy-derived tyrosine decarboxylase comprises the nucleic acid molecule represented by SEQ ID NO.2.
9. A biomaterial, wherein the biomaterial is any one of the following:
   B1) a nucleic acid molecule encoding glucosyltransferase U8GT3;
   B2) an expression cassette comprising the nucleic acid molecule described in B1); and
   B3) a recombinant vector comprising the nucleic acid molecule described in B1), or a recombinant vector comprising the expression cassette described in B2);
   preferably,
   the glucosyltransferase U8GT3 comprises an amino acid sequence represented by SEQ ID NO.10;
   more preferably,
   the glucosyltransferase U8GT3 comprises a nucleic acid molecule represented by SEQ ID NO.4.
10. A whole-cell catalyst, comprising the strain according to any one of items 5 to 7 or the biomaterial according to any one of items 8 to 9.
11. A method for producing salidroside and/or tyrosol using yeast, wherein the salidroside and/or tyrosol are produced by fermentation using the strain according to any one of items 5 to 8, the biomaterial according to any one of items 9 to 10, or the whole-cell catalyst according to item 11;
   preferably,
   the method comprises: adding galactose when the OD₆₀₀ is 8 to 15 during fermentation;
   more preferably, the concentration of galactose is 1-4 g/L;
   more preferably, the fermentation time is 90-120h;
   most preferably, the strain is *Saccharomyces cerevisiae.*
12. A method for producing salidroside, comprising the following steps:
   synthesizing tyrosol using a carbon source; and
   synthesizing salidroside using tyrosol;
   wherein in the step of synthesizing tyrosol using a carbon source, benzoate dehydrogenase and phenylpyruvate decarboxylase are used to synthesize tyrosol, and
   in the step of synthesizing salidroside using tyrosol, glucosyltransferase is used to synthesize salidroside,
   preferably,
   the glucosyltransferase is derived from *Rhodiola rosea*;
   more preferably,
   the glucosyltransferase comprises a nucleic acid molecule represented by SEQ ID NO.4; and
   the glucosyltransferase U8GT3 comprises an amino acid sequence represented by SEQ ID NO.10.
13. The method according to item 12,
   the method further comprises: synthesizing tyrosol by using a poppy-derived tyrosine decarboxylase and/or tyrosine decarboxylase mutants in the step of synthesizing tyrosol using a carbon source;
   preferably,
   the tyrosine decarboxylase mutant comprises an amino acid sequence represented by SEQ ID NO.7;
   the poppy-derived tyrosine decarboxylase comprises an amino acid sequence represented by SEQ ID NO.8; and
   the glucosyltransferase U8GT3 comprises an amino acid sequence represented by SEQ ID NO.10;
   more preferably,
   the tyrosine decarboxylase mutant comprises a nucleic acid molecule represented by SEQ ID NO.1;
   the poppy-derived tyrosine decarboxylase comprises a nucleic acid molecule represented by SEQ ID NO.2; and
   the glucosyltransferase U8GT3 comprises a nucleic acid molecule represented by SEQ ID NO.4.

### Effect of the present application

(1) The present application achieves the decoupling of microbial growth and product synthesis, reduces the metabolic pressure of the target pathway on the engineered bacteria, and further improves production efficiency while increasing cell density.
(2) The present application introduces the tyrosine decarboxylase mutant TYDC^{Y350F}, designs and constructs a new precursor synthesis pathway, pulls the metabolic flow toward the synthesis of the target product salidroside, and improves the economic benefits of fermentation production of salidroside.
(3) The yeast engineered bacteria of the present application can directly synthesize salidroside by metabolizing glucose, methanol, galactose, glycerol or trehalose, etc. Compared with traditional plant extraction, it is not restricted by climate and geographical conditions, is environmentally friendly, and the fermentation substrate is common and inexpensive, meeting the strategic needs of the country's green biomanufacturing. And there are no potential pathogenic factors, the quality and safety of the product are guaranteed, and it is suitable for the fields of medicine, cosmetics, etc.

### BRIEF DESCRIPTION OF THE DRAWINGS

Figure 1 is a diagram showing the fermentation results of a strain synthesizing salidroside de *novo*;
Figure 2 is a liquid chromatograms of salidroside standard and salidroside produced by the recombinant strain under the same conditions.

### DETAIL DESCRIPTION

The present application is further described below in conjunction with Examples. It should be understood that the Examples are only used to further describe and illustrate the present application and are not used to limit the present application.

Unless otherwise defined, technical and scientific terms used in this specification have the same meanings as commonly understood by those skilled in the art. Although methods and materials similar or equivalent to those described herein can be used in experiments or applications, the materials and methods are described below. In case of conflict, the present specification, including definitions, will control and, in addition, the materials, methods, and examples are illustrative only and not limiting. The present application is further described below in conjunction with specific Examples, but is not intended to limit the scope of the present application.

### <Enzymes>

Benzoate dehydrogenase is derived from *Saccharomyces cerevisiae* and can catalyze the conversion of prephenic acid to 4-hydroxyphenylpyruvate, thereby participating in tyrosine synthesis. Overexpression of benzoate dehydrogenase can promote tyrosine synthesis and thus promote metabolic flow to salidroside.

Benzoate dehydrogenase in the present application is referred to as TYR1.

Phenylpyruvate decarboxylase is derived from *Saccharomyces cerevisiae* and catalyzes the decarboxylation of phenylpyruvic acid to phenylacetaldehyde, which is the first specific step of the Ehrlich pathway. Overexpression of this phenylpyruvate decarboxylase can increase the level of tyrosol in *Saccharomyces cerevisiae* and thus increase the accumulation of salidroside precursor substances.

Phenylpyruvate decarboxylase in the present application is referred to as ARO10. Glucosyltransferase is derived from *Rhodiola rosea.* Under the catalysis of this enzyme, uri-dine diphosphate glucose (UDPG) serves as a glycosylation donor, and tyrosol is glycosylated to produce salidroside.

The glucosyltransferase in the present application is UDP-glucosyltransferase, referred to as U8GT3 or RvU8G3.

Tyrosine decarboxylase is a pyridoxal phosphate (PLP)-dependent decarboxylase derived from poppy. Tyrosine is catalyzed by the enzyme to generate tyramine, which is then catalyzed by monoaminoxidase to generate 4-hydroxyphenylacetaldehyde (4-HPAA); 4-HPAA is reduced by 4-hydroxybenzyl alcohol dehydrogenase (areB) to synthesize aglycone tyrosol of salidroside.

In one embodiment of the present application, the tyrosine decarboxylase is a poppy-derived tyrosine decarboxylase. In one embodiment of the present application, the tyrosine decarboxylase is a tyrosine decarboxylase mutant of the present application.

The tyrosine decarboxylase mutant of the present application, referred to as TYDC^{Y350F}, refers to a mutant in which the original tyrosine at position 350 is mutated to phenylalanine, and its function is changed from catalyzing the decarboxylation of tyrosine to form tyramine to catalyzing the direct generation of 4-hydroxyphenylacetaldehyde from tyrosine.

### <DNA>

In the present application, the glucosyltransferase is derived from *Rhodiola rosea.*

In the present application, the GeneBank number of the glucosyltransferase encoding gene is AUI41117.

In some embodiments of the present application, the amino acid sequence of the glucosyltransferase U8GT3 is shown as SEQ ID NO.10.

In some embodiments of the present application, the nucleic acid molecule of the glucosyltransferase U8GT3 is shown as SEQ ID NO.4.

In the present application, the tyrosine decarboxylase is derived from poppy (*Papaver somniferum*).

In some embodiments of the present application, the amino acid sequence of the poppy-derived tyrosine decarboxylase is shown as SEQ ID NO.8.

In some embodiments of the present application, the nucleic acid molecule of the poppy-derived tyrosine decarboxylase is shown as SEQ ID NO.2. In the present application, the tyrosine decarboxylase mutant is a mutant in which the tyrosine at position 350 is mutated to phenylalanine.

In some embodiments of the present application, the amino acid sequence of the tyrosine decarboxylase mutant is shown as SEQ ID NO.7.

In some embodiments of the present application, the nucleic acid molecule of the tyrosine decarboxylase mutant is shown as SEQ ID NO.1.

In some embodiments of the present application, the tyrosine decarboxylase is derived from *Arabidopsis thaliana,* wherein the amino acid sequence of the tyrosine decarboxylase derived from *Arabidopsis thaliana* is shown as SEQ ID NO.9.

In some embodiments of the present application, the nucleic acid molecule of the tyrosine decarboxylase derived from *Arabidopsis thaliana* is shown as SEQ ID NO.3.

In the present application, the GeneBank number of the phenylpyruvate decarboxylase encoding gene is 851987.

In some embodiments of the present application, the amino acid sequence of the phenylpyruvate decarboxylase ARO10 is shown as SEQ ID NO.11.

In some embodiments of the present application, the nucleic acid molecule of phenylpyruvate decarboxylase ARO10 is shown as SEQ ID NO.5.

In the present application, the GeneBank number of the benzoate dehydrogenase encoding gene is 852464.

In some embodiments of the present application, the amino acid sequence of the benzoate dehydrogenase TYR1 is shown as SEQ ID NO.12.

In some embodiments of the present application, the nucleic acid molecule of benzoate dehydrogenase TYR1 is shown as SEQ ID NO.6.

### <Biomaterials>

The present application provides a biomaterial, wherein the biomaterial is any one of the following:
A1) a nucleic acid molecule encoding a poppy-derived tyrosine decarboxylase and/or a nucleic acid molecule encoding a tyrosine decarboxylase mutant;
A2) an expression cassette comprising the nucleic acid molecule described in A1):
   A3) a recombinant vector comprising the nucleic acid molecule described in A1), or a recombinant vector comprising the expression cassette described in A2); and
   A4) A recombinant microorganism comprising the nucleic acid molecule described in A1), or a recombinant microorganism comprising the expression cassette described in A2), or a recombinant microorganism comprising the recombinant vector described in A3), preferably the recombinant microorganism is yeast.

The poppy-derived tyrosine decarboxylase is described above.

The tyrosine decarboxylase mutants are described above.

The present application provides a biomaterial, wherein the biomaterial is any one of the following:
B1) a nucleic acid molecule encoding a glucosyltransferase U8GT3, wherein the glucosyltransferase is derived from *Rhodiola rosea*;
B2) an expression cassette comprising the nucleic acid molecule described in B1);
B3) a recombinant vector comprising the nucleic acid molecule described in B1), or a recombinant vector comprising the expression cassette described in B2); and
B4) a recombinant microorganism comprising the nucleic acid molecule described in B1), or a recombinant microorganism comprising the expression cassette described in B2), or a recombinant microorganism comprising the recombinant vector described in B3), preferably the recombinant microorganism is yeast.

The glucosyltransferase U8GT3 is described above.

### <Production Process>

The present application provides the use of the above-mentioned glucosyltransferase U8GT3 and/or the above-mentioned poppy-derived tyrosine decarboxylase and/or the above-mentioned tyrosine decarboxylase mutant in increasing the yield of salidroside synthesized by using yeast.

The present application provides the use of the above-mentioned poppy-derived tyrosine decarboxylase and/or the above-mentioned tyrosine decarboxylase mutant in increasing the yield of tyrosol synthesized by using yeast.

The present application provides the use of the above-mentioned biomaterial in the synthesis of tyrosol.

The present application provides a strain for producing salidroside, comprising heterologously expressing one or more genes of a) to c) as follows in yeast;
a) a gene encoding glucosyltransferase U8GT3;
b) a gene encoding a poppy-derived tyrosine decarboxylase; and
c) a gene encoding a tyrosine decarboxylase mutant;

The glucosyltransferase U8GT3, poppy-derived tyrosine decarboxylase and tyrosine decarboxylase mutants are described above.

In some embodiments of the present application, a gene encoding phenylpyruvate decarboxylase ARO10 and/or a gene encoding benzoate dehydrogenase TYR1 is further heterologously expressed in yeast.

Phenylpyruvate decarboxylase ARO10 and benzoate dehydrogenase TYR1 are described above.

The present application provides a whole-cell catalyst comprising the above-mentioned strain or the above-mentioned biomaterial.

The present application provides a method for producing salidroside and/or tyrosol by using yeast, the salidroside and/or tyrosol are produced by fermentation using the above strain, the above biomaterial or the above whole-cell catalyst.

The present application provides a method for producing salidroside, comprising the following steps: synthesizing tyrosol using a carbon source; and synthesizing salidroside using tyrosol; wherein, in the step of synthesizing tyrosol using a carbon source, benzoate dehydrogenase and phenylpyruvate decarboxylase are used to synthesize tyrosol, and in the step of synthesizing salidroside using tyrosol, glucosyltransferase is used to synthesize salidroside.

The present application explores the effects of tyrosine decarboxylase TYDC and tyrosine decarboxylase mutant TYDC^{Y350F} on precursor synthesis. The tyrosine decarboxylase mutant TYDC^{Y350F} can greatly increase the accumulation of the precursor 4-HPAA, while tyrosine decarboxylase TYDC converts tyramine into a byproduct tyrosine, reducing the synthesis of the precursor.

In some embodiments of the present application, the method for producing salidroside comprises the following steps: synthesizing tyrosol using a carbon source; and synthesizing salidroside using tyrosol; wherein, in the step of synthesizing tyrosol using a carbon source, benzoate dehydrogenase, phenylpyruvate decarboxylase and poppy-derived tyrosine decarboxylase and/or a tyrosine decarboxylase mutant are used to synthesize tyrosol, and in the step of synthesizing salidroside using the tyrosol, glucosyltransferase is used to synthesize salidroside.

The tyrosine decarboxylase TYDC^{Y350F} mutant refers to a mutant in which the tyrosine at position 350 is mutated to phenylalanine, and its function is changed from catalyzing the decarboxylation of tyrosine to form tyramine to catalyzing the direct generation of 4-hydroxyphenylacetaldehyde from tyrosine.

The glucosyltransferase U8GT3, poppy-derived tyrosine decarboxylase and tyrosine decarboxylase mutants are described above.

The phenylpyruvate decarboxylase ARO10 and benzoate dehydrogenase TYR1 are described above.

In some embodiments of the present application, yeast is used as the starting strain, and the yeast engineering bacteria are constructed by introducing the above-mentioned nucleic acid molecules encoding benzoate dehydrogenase TYR1, phenylpyruvate decarboxylase ARO10, tyrosine decarboxylase TYDC^{Y350F} and glucosyltransferase U8GT3, wherein the introduced genes are all controlled by a galactose-inducible promoter.

In some embodiments of the present application, the method for producing salidroside is to use engineered bacteria for fermentation.

In some embodiments of the present application, the strain is an optional yeast suitable for the system of the present application, such as *Saccharomyces cerevisiae, Candida mycoderma, Rhodothece glutinis, Pichia pastoris,* brewer's yeast, *Candida mycoderma,* wine yeast, *Saccharomyces pastorianus,* aroma producing yeast and *Geotrichum candidum,* etc., preferably, *Saccharomyces cerevisiae.*

In some embodiments of the present application, galactose is added during fermentation culture when OD₆₀₀ is 8-15; for example, the OD₆₀₀ can be 8, 9, 10, 11, 12, 13, 14, 15 or any range therebetween. In some embodiments of the present application, the concentration of galactose is 1-4 g/L; for example, the concentration of galactose can be 1 g/L, 2 g/L, 3 g/L, 4 g/L or any range therebetween.

In some embodiments of the present application, the fermentation time is 90-120 h; for example, the fermentation time is 90 h, 95 h, 100 h, 105 h, 110 h, 115 h, 120 h or any range therebetween.

The present application provides an enzyme combination, comprising: benzoate dehydrogenase, phenylpyruvate decarboxylase, and glucosyltransferase; or comprising: benzoate dehydrogenase, phenylpyruvate decarboxylase, glucosyltransferase and tyrosine decarboxylase.

The enzyme combination refers to the combination of benzoate dehydrogenase, phenylpyruvate decarboxylase, and glucosyltransferase in the present application; or the combination of benzoate dehydrogenase, phenylpyruvate decarboxylase, glucosyltransferase and tyrosine decarboxylase in the present application. In the present application, the enzyme combination is intended to indicate a combination of enzymatic functions, that is, it can be a physical mixture of three enzymes or four enzyme proteins, for example, it can be a direct mixture of purchased pure enzymes, or a direct mixture of crude enzyme solutions or purified enzymes produced by genetic recombination expression using molecular biological methods. The combination of enzymes can be a fusion protein formed by fusing three or four enzymes on a three-dimensional protein structure, as long as they can each perform their corresponding functions. Similarly, it can be a simple mixture of a fusion protein of any two enzymes and another two enzymes, or a simple mixture of a fusion protein of any three enzymes and another enzyme, or a simple mixture of a fusion protein of any two enzymes and another two enzymes. Alternatively, it may be a simple mixture of a fusion protein of any two enzymes and another enzyme, or it may be a fusion protein of any three enzymes, or it may be a fusion protein of any three enzymes.

Likewise, in the present application, there is no limitation on the process of producing enzymes by gene recombination expression using molecular biological methods, and any known methods may be used. In the present application, the same host and one plasmid can be used to simultaneously express four enzymes or fusion proteins of three enzymes, or different hosts can be used to produce one or two of the enzymes or fusion proteins of the enzymes.

In some embodiments of the present application, the combination is a fusion protein formed by benzoate dehydrogenase, phenylpyruvate decarboxylase, and glucosyltransferase; or
the combination is a combination of a fusion protein formed by benzoate dehydrogenase and phenylpyruvate decarboxylase with glucosyltransferase; or
the combination is a fusion protein formed by benzoate dehydrogenase, phenylpyruvate decarboxylase, glucosyltransferase and tyrosine decarboxylase; or

The combination is a combination of a fusion protein formed by benzoate dehydrogenase, phenylpyruvate decarboxylase and glucosyltransferase with tyrosine decarboxylase; or
the combination is a combination of a fusion protein formed by benzoate dehydrogenase and phenylpyruvate decarboxylase with glucosyltransferase and tyrosine decarboxylase; or
the combination is a combination of a fusion protein formed by benzoate dehydrogenase and phenylpyruvate decarboxylase with a fusion protein formed by glucosyltransferase and tyrosine decarboxylase.

In some embodiments of the present application, the combination is a mixture of a fermentation crude enzyme solution of benzoate dehydrogenase, a fermentation crude enzyme solution of phenylpyruvate decarboxylase, and a fermentation crude enzyme solution of glucosyltransferase.

In some embodiments of the present application, the combination is a mixture of a fermentation crude enzyme solution of benzoate dehydrogenase, a fermentation crude enzyme solution of phenylpyruvate decarboxylase, a fermentation crude enzyme solution of glucosyltransferase, and a fermentation crude enzyme solution of tyrosine decarboxylase.

In some embodiments of the present application, the combination is a fusion protein formed by benzoate dehydrogenase, phenylpyruvate decarboxylase, glucosyltransferase and tyrosine decarboxylase; or a combination of a fusion protein of benzoate dehydrogenase and phenylpyruvate decarboxylase with glucosyltransferase and tyrosine decarboxylase; or a combination of a fusion protein of benzoate dehydrogenase and phenylpyruvate decarboxylase with a fusion protein of glucosyltransferase and tyrosine decarboxylase.

The present application provides a whole-cell catalyst comprising the above-mentioned genetically engineered bacteria or a combination of the above-mentioned genetically engineered bacteria.

The present application provides a method for producing salidroside, comprising the above-mentioned enzyme combination.

### EXAMPLES

### Example 1 Cloning of genes required for salidroside synthesis.

The plant- or microbial-derived enzymes required for the synthesis pathway of salidroside were determined from different plant sources through the National Center for Biotechnology Information (NCBI) (https://www.ncbi.nlm.nih.gov/) and literature searches, and the corresponding amino acid sequences or gene sequences were found.

The obtained gene sequence was codon optimized by the codon optimization algorithm developed by GenScript Biotech Co., Ltd. and according to the corresponding yeast host, as shown in the sequence listing, wherein the nucleic acid molecule of the tyrosine decarboxylase TYDC^{Y350F} is shown as SEQ ID NO.1, and the nucleic acid molecule of the glucosyltransferase U8GT3 is shown as SEQ ID NO.4. And the universal plasmid pESC series with galactose promoter (pGAL1 or pGAL10) were directly synthesized to obtain the recombinant plasmid with the target gene, see Table 1 for details.

**Table 1 Plasmids used for strain construction**

| Plasmid name | Genotype |
|---|---|
| pESC-LEU- *RvU8GT3* | 2µ; *LEU2; AmpR; GAL1p- RvU8GT3-ADH1t* |
| *pESC*-TRP-*TYDC^{Y350F}* | 2µ; *TRP1; AmpR; GAL1p-TYDC^{Y350F}-CYC1t* |
| *pESC-TRP-TYDC* | 2µ; *TRP1; AmpR; GAL1p-TYDC-CYC1t* |
| *pESC-TRP-AtTYDC* | *2µ; TRP1; AmpR; GAL1p-TYDC-CYC1t* |
| *pESC-URA-ARO10-TYR1* | 2µ; *URA3; AmpR; GAL1p-1YR1-CYC1t; GAL10p-ARO10-ADH1t* |

### Example 2 Construction of the engineered bacteria.

### 2.1 Experimental Materials

The experimental materials used in this example were primers 1-F and 1-R, and primers 2-F and 2-R.

Primer 1-F: ttaacgtcaaggagaaaaaaccccggatccatggtatcagaggataagattgagc (SEQ ID NO.13)

Primer 1-R: tagctagccgcggtaccaagcttactcgagttatgtatttcttttttcagcggcc (SEQ ID NO.14)

Primer 2-F: tccttgtaatccatcgatactagtgcggccgcctattttttatttcttttaagtgccgct (SEQ ID NO.15)

Primer 2-R: tcgaattcaaccctcactaaagggcggccgcatggcacctgttacaattgaaaag (SEQ ID NO.16)

### 2.2 Construction of pESC-TYR1-Aro10 recombinant plasmid

### 1) Construction of vector fragment

According to the enzyme digestion system shown in Table 2, the vector pESC was treated with the endonucleases NotI and BamHI purchased from NEB Company at the same time, and the prepared enzyme digestion system was placed in water bath at 37°C for 1 hour. The backbone and promoter after enzyme digestion were purified and recovered using a gel recovery kit purchased from Thermo Fisher Scientific to obtain purified Backbone1 fragment and *Gal1-Gal10* dual promoter fragment.

**Table 2 Enzyme digestion system**

| Component | Volume |
|---|---|
| NotI/BamHI | 1 µL |
| Vector pESC | 1 µg |
| 10× NEB Buffer | 1 µL |
| ddH₂O | Supplement to 10 µL |

### 2) Amplification and recovery of target genes

**Table 3 PCR reaction system (2× Phanta Flash Master Mix**

| Component | Volume |
|---|---|
| Genomic DNA template/plasmid template | 1 µL |
| Forward primer | 2 µL |
| Reverse primer | 2 µL |
| 2× Phanta Flash Master Mix | 25 µL |
| ddH₂O | Supplement to 50 µL |

**Table 4 PCR reaction procedure (2× Phanta Flash Master Mix)**

| Step | Temperature | Time | Number of cycles |
|---|---|---|---|
| Pre-denaturation | 98 °C | 30 s | 1 |
| Denaturation | 98 °C | 10 s | 35 |
| Annealing | (Tm-5) °C | 5 s | |
| Extension | 72 °C | 4-5 s/kb | |
| Post extension | 72 °C | 1 min | 1 |

2× Phanta Flash Master Mix purchased from Vazyme Biotech Co., Ltd. was used as the amplification enzyme, and primer 1-F, primer 1-R, primer 2-F, and primer 2-R were used to clone the genes of *TYR1* and *Aro10,* respectively. The PCR reaction system and reaction procedure were shown in Tables 3 and 4. The amplified genes *Aro10* and *TYR1* were DNA purified and recovered using a gel recovery kit purchased from Thermo Fisher Scientific.

### 3) In vitro multi-fragment ligation by SOE-PCR

**Table 5 SOE-PCR reaction system (2× Phanta Flash Master Mix)**

| Component | Volume |
|---|---|
| Gene fragment *Aro10* | 1 µL |
| Gene fragment *Gal1-Gal10* | 1 µL |
| Gene fragment *TYR1* | 1 µL |
| Forward primer | 2 µL |
| Reverse primer | 2 µL |
| 2× Phanta Flash Master Mix | 25 µL |
| ddH₂O | Supplement to 50 µL |

The purified *TYR1, Aro10* and *Gal1-Gal10* promoter fragments were ligated and amplified using SOE-PCR. The obtained T*YR1, Aro10* and *Gal1-Gal10* promoter fragments were used as templates, 2× Phanta Flash Master Mix purchased from Vazyme Biotech Co., Ltd. was used as an amplification enzyme, and primers 1-F and 2-R were used for PCR, respectively. The PCR reaction system was shown in Table 5, and the PCR reaction procedure was shown in Table 4. The gene *TYR1-Gal1-Gal10-Aro10* obtained by ligation amplification was DNA purified and recovered using a gel recovery kit purchased from Thermo Fisher Scientific.

### 4) Gibson ligation

The *TYR1-Gal1-Gal10-Aro10* fragment was ligated to the Backbone1 fragment by the Gibson ligation method to construct the recombinant plasmid pESC-TYR1-Aro10; the NEBuilder^{®} HiFi DNA Assembly Master Mix used was from NEB, and the ligation reaction system was shown in Table 6, in which the ratio of vector to inserted gene was 1:2. The prepared ligation system was placed in a water bath at 50°C and taken out after 60 minutes, and the ligation product was transformed into *Escherichia coli* DH5α.

**Table 6 Gibson ligation system**

| Component | Volume |
|---|---|
| Inserted gene | 0.03-0.2 pmol |
| Vector | 0.03-0.2 pmol |
| NEBuilder^{®} HiFi DNA Assembly Master Mix | 10 µL |
| ddH₂O | Supplement to 20 µL |

### 5) Chemical transformation of E. coli

The *E. coli* competent cells DH5α were taken out from the ultra-low temperature refrigerator and placed on ice to thaw. 10 µL of Gibson ligation product was added to the thawed competent cells, flicked to mix evenly, placed on ice for 30 minutes, heat shocked in a water bath at 42°C for 90 seconds, taken out and placed on ice for 2 minutes. 200 µL of LB liquid culture medium was added and cultured at 37°C and 220 rpm for 60 minutes. In the clean bench, an appropriate amount of bacterial liquid was taken and coated on LB solid culture medium comprising ampicillin (100 mg/L), and cultured upside down in a constant temperature incubator at 37°C overnight.

### 6) Sequencing verification

After a single colony grown on the above plate, the above recombinant plasmids were screened and identified using the colony PCR method. 2× Rapid Taq Master Mix purchased from Vazyme Biotech Co., Ltd. was used as the amplification enzyme, and the colony PCR reaction system was shown in Table 7. After the preparation was completed, a single colony of *E. coli* was picked up with a sterile pipette tip and placed in 10 µL of sterile water in a clean bench, fully dissolved, and taken 1 µL as a template, added to the colony PCR reaction system, and performed gene amplification according to the reaction procedure in Table 8.

**Table 7 PCR reaction system (2× Rapid Taq Master Mix)**

| Component | Volume |
|---|---|
| template | 1 µL |
| Forward primer | 0.5 µL |
| Reverse primer | 0.5 µL |
| 2× Rapid Taq Master Mix | 10 µL |
| ddH₂O | Supplement to 20 µL |

**Table 8 PCR reaction procedure (2× Rapid Taq Master Mix)**

| Step | Temperature | Time | Number of cycles |
|---|---|---|---|
| Pre-denaturation | 95 °C | 3 min | 1 |
| Denaturation | 95 °C | 15 s | 30 |
| Annealing | (Tm-5) °C | 15 s | |
| Extension | 72 °C | 15 s/kb | |
| Post extension | 72 °C | 5 min | 1 |

The PCR product was verified by agarose gel electrophoresis, and a single colony comprising the target band was selected, inoculated into LB medium comprising ampicillin (100 mg/L) and cultured overnight at 37°C and 220 rpm. The corresponding plasmid was extracted using a plasmid extraction kit purchased from Tiangen Biotech (Beijing) Co., Ltd. and delivered to GENEWIZ, Inc. for gene sequencing verification.

### 2.2 Construction of SAD1, SAD2, SAD3, SDA4 and SAD5 strains

Using the genes in Table 9 and the integration plasmids as templates, the linearized fragments for yeast genome integration were amplified by PCR (the reaction system and procedures were shown in Tables 3 and 4). The target gene fragments comprising the homology arms of the integration site were obtained by agarose gel electrophoresis and gel recovery. Then, yeast transformation was performed according to the method described in Example 1. The transformed yeast was homologous recombined with the chassis host genome by using the homology arms of 40 bp on the left and right of the integration site on the target fragment, and the expression cassette comprising the fusion gene was integrated into the target site of the yeast chassis genome. ARO10 and TYR1 were integrated into the defective position of *Saccharomyces cerevisiae* UAR3 (recorded as CEN.PK2-ΔURA3::ARO10-TYR1, and the yeast was named SAD1); on the basis of this chassis bacteria (SAD1), U8GT3 was integrated into the GAL80 site (recorded as SAD2-ΔGAL80::U8GT3, the yeast was named SAD2); on the basis of this chassis bacteria (SAD2), TYDC^{Y350F} was integrated into the HIS3 site (recorded as SAD2-ΔHIS3::TYDC^{Y350F}, the yeast was named SAD3); on the basis of this chassis bacteria (SAD2), poppy-derived tyrosine decarboxylase (abbreviated as TYDC) was integrated into the HIS3 site (recorded as SAD2-ΔHIS3::TYDC, the yeast was named SAD4); on the basis of this chassis bacteria (SAD2), Arabidopsis thaliana-derived tyrosine decarboxylase (abbreviated as AtTYDC) was integrated into the HIS3 site (recorded as SAD2-ΔHIS3::AtTYDC, the yeast was named SAD5).

### PCR verification of yeast transformants

The transformed coated plate was inverted and cultured at 30°C for 2-3 days. After a single colony grew on the plate, a single colony grown on the solid plate was picked and inoculated into SC liquid culture medium, and cultured at 30°C and 220 rpm overnight. The genome of the yeast overnight culture was extracted using the yeast genome extraction kit and used as a template for PCR verification. The PCR method and procedure were shown in Tables 3 and 4. For the transformants on the plate, cells were broken at selected spots, and two primers on the genome (see Table 10 for details) were selected to use PCR to verify whether the target gene was integrated into the genome. If the band size was correct and the sequencing results were correct, it indicated that the genome integration was successful.

**Table 9 Primers used for genome integration of heterologous genes**

| Primer name | Primer sequence (5' to 3') |
|---|---|
| URA3-*ARO10*-*TYR1*-F | ttgtacaactgtgctaaacagacttaaaaaagtaataattgagcgacctcatgctatac (SEQ ID NO.17) |
| *URA3-ARO10-TYR1-*R | Ctattggagtgatcaaaaaaaacttcaattagcgtgataccttcgagcgtcccaaaacc (SEQ ID NO.18) |
| GAL80- *RvU8G3-*F | acactcgcgagaaccaaaacaaggatcccctaaacccagt atccgctctaaccgaaaag (SEQ ID NO.19) |
| GAL80- *RvU8G3*-R | aagcgatccgtagtcctatccgggtaacactcatcgtccg cttcgagcgtcccaaaacc (SEQ ID NO.20) |
| HIS3- *TYDC^{Y350F}* -F | tcgtgttcatgcagatagataacaatctatatgttgataa gagcgacctcatgctatac (SEQ ID NO.21) |
| HIS3- *TYDC^{Y350F}* -R | cataaaagaccgtgtgatggcttggcatggcgatttcatt cttcgagcgtcccaaaacc (SEQ ID NO.22) |

**Table 10 Primers used for genome verification of heterologous genes**

| Primer name | Primer sequence (5' to 3') |
|---|---|
| Conf-URA-F | aaagctacatataaggaacgtgct (SEQ ID NO.23) |
| Conf-URA-R | caggctgggaagcatatttgag (SEQ ID NO.24) |
| Conf-GAL80-F | ttcatttaccggcgcactctcgcc (SEQ ID NO.25) |
| Conf-GAL80-R | taaataagaacacccgcatgcac (SEQ ID NO.26) |
| Conf-HIS3-F | agagcagaaagccctagtaaagc (SEQ ID NO.27) |
| Conf-HIS3-R | aacatcgttggtaccattgggc (SEQ ID NO.28) |

### Example 3

The engineered bacteria of *Saccharomyces cerevisiae* that synthesize salidroside *de novo* were fermented in shake flasks. SAD1, SAD2, SAD3, SDA4, and SAD5 were all cultured under the same conditions. First, a single colony was streaked on a YPD plate and then inoculated into 50 mL YPD liquid culture medium. At 30°C and 250 rpm, SAD1, SAD2, SAD3, SDA4, and SAD5 were cultured until the OD₆₀₀ of bacterial reached about 6, and then transferred to 20 mL YPD liquid culture medium (glucose concentration is 2%) with an initial OD₆₀₀=0.4. When the OD₆₀₀ reached about 12, the concentration of added galactose needed to be stabilized at 2g/L, and the entire fermentation cycle was maintained for about 120h.

**Table 11 The yield of salidroside produced by various strains in shake flasks**

| Naming of bacterial strains | Gene modification | Yield of salidroside (mg/L) | Yield of tyrol (mg/L) |
|---|---|---|---|
| SAD1 | CEN.PK2- ΔURA3:: ARO 10-TYR1 | 0 | 921.2 |
| SAD2 | SAD1-ΔGAL80:: RvU8G3 | 608.2 | 23 |
| SAD3 | SAD2- ΔHIS3:: TYDC^{Y350F} | 803.1 | 13 |
| SAD4 | SAD2- ΔHIS3:: TYDC | 680.2 | 32 |
| SAD5 | SAD2- ΔHIS3:: AtTYDC | 656.3 | 28 |

1 mL of the fermentation supernatant was taken and filtered using a 0.22 µm organic filter head. 10 µL of the sample was then separated using a Shimadzu LC-20A (Shimadzu), HyPURITYTM C18 HPLC (250 mm×4.6 mm, 3 µm, Ultimate LP-C18) column. Wherein, mobile phase Ais 0.1% formic acid aqueous solution, and mobile phase B is 0.1% acetonitrile solution. The intermediates and products were separated by isocratic elution using 8% mobile phase B and 92% mobile phase A, and salidroside was detected at a wavelength of 224 nm using an ultraviolet detector.

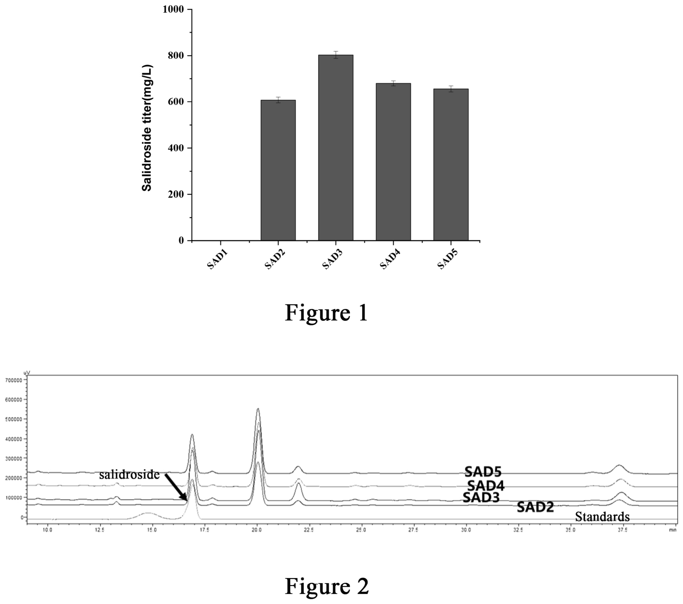

The results are shown in Figures 1 and 2. The production of salidroside by fermentation with the SAD3 strain, SDA4 and SAD5 strains shows that the salidroside production can reach 803.1 mg/L when TYDC^{Y350F} and RvU8G3 are heterologously expressed in the strain. Compared with SDA4 heterologously expressing AtTYDC and RvU8G3 from *Arabidopsis thaliana* and SAD5 heterologously expressing TYDC and RvU8G3 from poppy, the yield is increased by 18% and 22%, greatly improving the production of salidroside.

Although the present application has been disclosed as above by way of Examples, it is not intended to limit the present application. Any person having ordinary knowledge in the technical field may make some changes and modifications without departing from the spirit and scope of the present application. Therefore, the protection scope of the present application shall be determined by the scope of the patent application attached hereto.

### Sequence Listing

| I Sequence No. | Sequence |
|---|---|
| SEQ ID NO.1 | atgggttctttgccaaccaacaaccttgaatctatttccttatgctctcaaaacccattagacccagacgaattcagaagacaaggt cacatgatcattgatttcttggctgattactacaagaacgttgaaaagtacccagtcagatcccaagtcgaaccaggctacttaaa gaagagattgccagagtctgctccatacaacccagaatccatcgaaaccatcttggaagatgtcacaaacgatatcatcccagg tttgacccactggcaatctccaaattactttgcttacttcccatcttctggttctatcgccggttttttaggtgaaatgttgtctaccggtt tcaatgttgtcggtttcaactggatgtctagtccagctgccactgaattggaatctattgttatgaactggttgggtcaaatgttgactt tgccaaaatctttcttattctcttctgatggttcctccggtggtggtggtgtcttgcaaggtactacttgtgaagctatcttgtgtactttg accgctgctagagacaagatgctgaataaaattggtagagaaaacattaacaagttggttgtctacgcttccgatcaaactcattg tgctttgcaaaaggccgctcaaattgctggtattaaccctaaaaatgttagagctatcaagacctctaaggctactaacttcggtttg agcccgaactctttgcaatccgccattctagccgatattgaatccggattggttcctttgttcttgtgcgctactgtcggtaccacttc aagcactgctgtcgatccaatcggtccattgtgtgctgttgccaagttgtacggtatctgggttcacatagatgctgcttatgccggt tccgcttgtatctgtccagaattccgtcactttattgacggtgttgaagacgctgactctttctccttgaacgctcacaagtggttcttc accaccttggactgttgttgtttgtgggtcaaggattccgactccttggtcaaagctttatctacctctccagaatttttgaagaacaa ggctaccgaatccaagcaagttattgactacaaggactggcaaatcgctttgtcacgtagattcagatccatgaagctatggttag ttttgagatcttacggtgttgctaacttgagaaccttcttgcgttctcatgtcaagatggctaagcatttccaaggtttgattggtatgg acaacagatacgaaatcgttgtcccaagaactttcgctatggtttgtttcagattaaagccaactgccatcttcaagcaaaagattgt tgacaacgactacattgaagaccaaacgaacgaagtcaacgtcaaattgttggaatctgttaacgcttctggtaagatctatatga ctcacgctgtcgttggtggtgtttacatgatcagatttgccgtaggtgccaccctcactgaagaaagacacgttactggtgcttgg aaggttgttcaagaacacactgatgcaatcttgggtgcttga |
| SEQ ID NO.2 | atgggaagccttccgactaataaccttgagagcatctcactgtgttcgcaaaatccacttgatccagatgaattcagaaggc aaggtcacatgattattgatttccttgctgattactacaaaaatgttgagaagtatccagttagaagccaagtcgagcccggtt atttgaagaaacggttacccgaatctgctccatacaatcctgaatccattgaaactattcttgaagatgtcacaaatgatatca tacctggtctaactcactggcaaagtccaaattactttgcttactttccttctagtggttccattgctggtttccttggagagatg ctaagtaccggatttaatgttgtcggcttcaattggatgtcatctccggctgcaactgagttggagagtattgttatgaattgg cttggccagatgcttacgcttcccaaatcatttctcttctcatcagatggaagttcaggaggaggcggtgttttgcaagggac tacttgtgaagccattttatgtactctaactgcagcgagagacaaaatgttgaacaaaattggtagagaaaatattaacaagt tggttgtttatgcttctgatcaaacccattgtgcacttcaaaaagctgctcagattgctgggattaatccgaagaacgtccgc gctattaaaacgtccaaggctacaaatttcggtctctctccaaattcacttcaatcggcaattcttgcagatatcgaatctggg ttagttccgttgttcctctgtgctaccgtcggaacaacttcttcaacagccgtagatcctattggccctctctgcgcggtggca aaactgtatggtatttgggttcatatcgatgcggcttatgctggaagtgcttgtatctgcccagagttcaggcacttcattgat ggtgtggaagatgcagattcatttagtctaaatgcacacaagtggttcttcactacattggattgttgttgtttatgggtgaaag actccgactcccttgttaaagcattatcaacaagtccagaatacttgaagaacaaagcaacggaatcaaaacaagttattga ttacaaagactggcaaatagctctcagcagaagattccgatccatgaaactctggttagtccttcgcagctacggggttgct aacttaagaaccttcctcaggagtcatgttaaaatggctaagcattttcaagggctcataggcatggataacaggtttgagat tgtggttccaagaacatttgccatggtgtgctttcgccttaaaccaactgcaattttcaagcaaaagatagtcgataatgatta cattgaagatcaaacaaatgaggtaaatgtgaaattgcttgaatcagtcaatgcgtccggcaagatatacatgactcatgct gttgtaggaggggtgtacatgattcggtttgccgttggggcaacactgacagaggaaagacatgtgactggggcgtgga aggtagttcaggagcatacagatgccatacttggtgcctag |
| SEQ ID NO.3 | atggaatttggtaccggcaacggctacagcaacgggaacggctacaccaatggaaacggccacaccaacggaaacgg caattacaacggcaatggccacgttaatggtaacggaaaagctaacggagccaaggtggtaaagatgaaaccaatggac agtgagttgctgagagagcaaggtcacataatggttgattttattgctgattattacaaaaaccttcaagactcgcctcaagat ttccctgttctgtcccaagttcagcctggttatctccgtgacatgttgcctgattcagcacctgaacgacctgaatcgttaaaa gaacttctcgacgatgtttcgaagaagataatgccggggataactcattggcaaagcccgagttactttgcatactatgcat caagcacgagtgtggctggattcttaggagagatgcttaacgccggcctgagcgtcgtcggttttacctggctcacttctcc tgccgctactgagctcgaaatcatcgttcttgattggcttgctaaattgctacaactccccgaccattttctctccacaggaaa |
| | aggaaatggaggaggagtaatccaagggacaggatgtgaagcagtgcttgtggtagtattagctgctagagacagaatt ctgaagaaagttggcaaaacattacttcctcaacttgttgtctatggctctgaccaaacccattctagtttccgaaaagcttgt ctgattggtgggatacatgaagaaaacattaggctgctcaaaactgattcttccacaaactatggaatgcctccagaatcac ttgaggaagctatttctcatgatctcgctaagggttttatccctttcttcatttgtgccactgttggcacaacgtcttcagcagcg gttgatcctttggtcccattggggaacatcgcaaagaaatatgggatatggttgcatgtggatgcagcttatgcagggaatg catgtatatgtccagaatatcgaaaatttattgacgggattgaaaacgcagactcctttaacatgaatgctcataaatggttatt tgctaatcaaacttgttcacctctttgggttaaggatcgatactctctcattgatgctctcaaaacaaatcccgagtatctagaa ttcaaggtttccaaaaaagatacggtcgtaaattataaagattggcaaatttctctctctcggagattcaggttctttataagtc catttgatgttgtatttgctggtcctattagtctaacaaacttatatgtacacgcatttgatttatacagatcactgaagttatggat ggttttacggctctatggttccgagaacttaagaaactttataagagaccatgtcaatctcgctaagcattttgaagattatgta gctcaagatccaagttttgaggttgtcactactcggtacttttcactcgtttgctttcgccttgcgccagttgacggcgatgaa gaccaatgtaacgaacgtaaccgtgaactgcttgcggctgttaactccactggcaagatattcatctctcacacggctctat ctggaaaattcgttttacgatttgctgttggtgcaccgttaacggaagagaagcatgtcactgaagcatggcagattataca gaaacatgcctccaaatttacacgcaacgaccattattaa |
| SEQ ID NO.4 | atgtctttgatcgaaaagccattgactgccattgaaaccagagaaaagccacacgctgtctgtattccttacccagctcaag gtcacatcaacccaatgatgcaattagccaagttgttgcaccactccggtttccacatcactttcgttcacactgaatacaact acgacagattagtcaaatctcaaggttctgcttgtgttgctggtttgccagacttcagattcgaagctatcccggacggtttgc cttctaccaatggtgatgttactcaagacattccattattgtcttcttctacttcaaagacctgtttgaagccattcaaggaattg ctcaaaagattgcaagataagtgtaaggaattaccagacgatgttccaccattgtcctgcattgtctccgacgctgctatgtc tttcaccatcgatgcttctgaagaattcggtgttccaattgctttgttgtggactgcttccgcctgtggtttcttgggttacaccc actacccatacttgatcgacagaggtgtcatcccattgaaggatgaaagccaattgaccaacggttacttggacatgtccat cgatggtattccatgtatggaaggtatcagattgcgtgatttgccatctttcttgagaaccaccgatctagacgacatgatgtt ctcttacattttgcacgaaattaagcaagtttccagaggttccgctatcatcttgaacactttcgaagctttggaccacgatgtt ttagattccttgtctaagatttaccaaaacgttattttgccagttggcccattgcatgtttcgttgaacaagatcccaaagcatta tccattgcaatctttgtcctctaacttgtggaaggatgacactgactgtatcccatggttaagttccaaggcctctaaatccgtt atatacgttaactttggttctatcactactgtctcaccaaaacaaatcgtcgagtttgcttggggtctggccaattccaagcac cctttcttatggattatcagaccagatttggtggctggtgaagcttctattattccacaagatttcatggacgaaactaagggta gaggtttgcttgctggttggtgtgaccaagaattggttttgaaccacccatccattggtggtttcttgactcactgcggctgga actccatcattgaatctatctctgccggtgtcccaaccgtctgttggccattctttgctgaacaacaaaccaactgttggttcg cttgtaagaagtggtgtatcggtatggaaatgcatactgatgtcaagagagacgaagttgacaaattattgagagaattgat ggaaggtgacaagggtgaagaattaaagagaaaggctacaaactggaagcgtttggctgaagaagccgtttcttcaact ggtctatccaccctaaactttagaactttggtcaaccaagtcttgttgtctaagaccaagcatatccgttga |
| SEQ ID NO.5 | atggcacctgttacaattgaaaagttcgtaaatcaagaagaacgacaccttgtttccaaccgatcagcaacaattccgtttg gtgaatacatatttaaaagattgttgtccatcgatacgaaatcagttttcggtgttcctggtgacttcaacttatctctattagaat atctctattcacctagtgttgaatcagctggcctaagatgggtcggcacgtgtaatgaactgaacgccgcttatgcggccga cggatattcccgttactctaataagattggctgtttaataaccacgtatggcgttggtgaattaagcgccttgaacggtatagc cggttcgttcgctgaaaatgtcaaagttttgcacattgttggtgtggccaagtccatagattcgcgttcaagtaactttagtgat cggaacctacatcatttggtcccacagctacatgattcaaattttaaagggccaaatcataaagtatatcatgatatggtaaa agatagagtcgcttgctcggtagcctacttggaggatattgaaactgcatgtgaccaagtcgataatgttatccgcgatattt acaagtattctaaacctggttatatttttgttcctgcagattttgcggatatgtctgttacatgtgataatttggttaatgttccacg tatatctcaacaagattgtatagtatacccttctgaaaaccaattgtctgacataatcaacaagattactagttggatatattcca gtaaaacacctgcgatccttggagacgtactgactgataggtatggtgtgagtaactttttgaacaagcttatctgcaaaact gggatttggaatttttccactgttatgggaaaatctgtaattgatgagtcaaacccaacttatatgggtcaatataatggtaaag aaggtttaaaacaagtctatgaacattttgaactgtgcgacttggtcttgcattttggagtcgacatcaatgaaattaataatgg gcattatacttttacttataaaccaaatgctaaaatcattcaatttcatccgaattatattcgccttgtggacactaggcagggc aatgagcaaatgttcaaaggaatcaattttgcccctattttaaaagaactatacaagcgcattgacgtttctaaactttctttgc aatatgattcaaatgtaactcaatatacgaacgaaacaatgcggttagaagatcctaccaatggacaatcaagcattattac acaagttcacttacaaaagacgatgcctaaatttttgaaccctggtgatgttgtcgtttgtgaaacaggctcttttcaattctctg ttcgtgatttcgcgtttccttcgcaattaaaatatatatcgcaaggatttttcctttccattggcatggcccttcctgccgcccta ggtgttggaattgccatgcaagaccactcaaacgctcacatcaatggtggcaacgtaaaagaggactataagccaagatt aattttgtttgaaggtgacggtgcagcacagatgacaatccaagaactgagcaccattctgaagtgcaatattccactagaa gttatcatttggaacaataacggctacactattgaaagagccatcatgggccctaccaggtcgtataacgacgttatgtcttg gaaatggaccaaactatttgaagcattcggagacttcgacggaaagtatactaatagcactctcattcaatgtccctctaaat |
| | tagcactgaaattggaggagcttaagaattcaaacaaaagaagcgggatagaacttttagaagtcaaattaggcgaattgg atttccccgaacagctaaagtgcatggttgaagcagcggcacttaaaagaaataaaaaatag |
| SEQ ID NO.6 | atggtatcagaggataagattgagcaatggaaagccacaaaagtcattggtataattggtctgggtgatatgggcctattat acgctaataaatttacagatgctggatggggtgttatatgttgtgatagggaagaatattatgatgaactgaaagaaaaatat gcctcagctaaattcgaactggtgaaaaatggtcatttggtatccaggcaaagcgactatattatctatagtgttgaagcatc caatattagtaagatcgtcgcaacgtatggaccatcttctaaggttggaacaattgttgggggtcaaacgagttgtaagctg ccggaaatcgaggctttcgaaaagtatttacccaaggactgcgacatcattaccgtgcattcccttcatgggcctaaagtta atactgaaggccaaccactagttattatcaatcacagatcacagtacccagaatcttttgagttcgttaattctgttatggcatg tttgaaaagtaagcaagtttatttgacatatgaagagcatgacaagattaccgctgatacacaagctgtgacacatgctgctt tcttaagtatgggatctgcgtgggcaaagataaagatttatccttggactctgggtgtaaacaaatggtacggtggcctaga aaatgtgaaagttaatatatcactaagaatctattcgaacaagtggcatgtttacgcaggattagccataacaaacccaagt gcacatcagcaaattcttcaatatgcaaccagtgcaacagaactatttagtttaatgatagataacaaagaacaagaacttac tgatagactattaaaagctaagcaatttgtatttggaaagcatactggtctcttactattggatgacacgattttagagaaatatt cgctatcaaaaagcagcattggtaacagcaacaattgcaagccagtgccgaattcacatttatcattgttggcgattgttgat tcgtggtttcaacttggtattgatccatatgatcatatgatttgttcgacgccattattcagaatattcctgggtgtgtccgaatat ctttttttaaaacctggcttattagaacagacaattgatgcagctatccatgataaatcattcataaaagatgatttagaatttgtt atttcggctagagaatggagctcggttgtttcttttgccaattttgatatatacaaaaagcaatttcagagtgttcaaaagttcttt gagccaatgcttccagaggctaatctcattggcaacgagatgataaaaaccattctgagtcattctagtgaccgttcggccg ctgaaaaaagaaatacataa |
| SEQ ID NO.7 | MGSLPTNNLESISLCSQNPLDPDEFRRQGHMIIDFLADYYKNVEKYPVRSQVE PGYLKKRLPESAPYNPESIETILEDVTNDIIPGLTHWQSPNYFAYFPSSGSIAGFL GEMLSTGFNVVGFNWMSSPAATELESIVMNWLGQMLTLPKSFLFSSDGSSGG GGVLQGTTCEAILCTLTAARDKMLNKIGRENINKLVVYASDQTHCALQKAAQI AGINPKNVRAIKTSKATNFGLSPNSLQSAILADIESGLVPLFLCATVGTTSSTAV DPIGPLCAVAKLYGIWVHIDAAYAGSACICPEFRHFIDGVEDADSFSLNAHKWF FTTLDCCCLWVKDSDSLVKALSTSPEFLKNKATESKQVIDYKDWQIALSRRFR SMKLWLVLRSYGVANLRTFLRSHVKMAKHFQGLIGMDNRYEIVVPRTFAMVC FRLKPTAIFKQKIVDNDYIEDQTNEVNVKLLESVNASGKIYMTHAVVGGVYMI RFAVGATLTEERHVTGAWKVVQEHTDAILGA* |
| SEQ ID NO.8 | MGSLPTNNLESISLCSQNPLDPDEFRRQGHMIIDFLADYYKNVEKYPVRSQVE PGYLKKRLPESAPYNPESIETILEDVTNDIIPGLTHWQSPNYFAYFPSSGSIAGFL GEMLSTGFNVVGFNWMSSPAATELESIVMNWLGQMLTLPKSFLFSSDGSSGG GGVLQGTTCEAILCTLTAARDKMLNKIGRENINKLVVYASDQTHCALQKAAQI AGINPKNVRAIKTSKATNFGLSPNSLQSAILADIESGLVPLFLCATVGTTSSTAV DPIGPLCAVAKLYGIWVHIDAAYAGSACICPEFRHFIDGVEDADSFSLNAHKWF FTTLDCCCLWVKDSDSLVKALSTSPEYLKNKATESKQVIDYKDWQIALSRRFR SMKLWLVLRSYGVANLRTFLRSHVKMAKHFQGLIGMDNRFEIVVPRTFAMVC FRLKPTAIFKQKIVDNDYIEDQTNEVNVKLLESVNASGKIYMTHAVVGGVYMI RFAVGATLTEERHVTGAWKVVQEHTDAILGA* |
| SEQ ID NO.9 | MEFGTGNGYSNGNGYTNGNGHTNGNGNYNGNGHVNGNGKANGAKVVKM KPMDSELLREQGHIMVDFIADYYKNLQDSPQDFPVLSQVQPGYLRDMLPDSA PERPESLKELLDDVSKKIMPGITHWQSPSYFAYYASSTSVAGFLGEMLNAGLSV VGFTWLTSPAATELEIIVLDWLAKLLQLPDHFLSTGKGNGGGVIQGTGCEAVL VVVLAARDRILKKVGKTLLPQLVVYGSDQTHSSFRKACLIGGIHEENIRLLKT DSSTNYGMPPESLEEAISHDLAKGFIPFFICATVGTTSSAAVDPLVPLGNIAKKY GIWLHVDAAYAGNACICPEYRKFIDGIENADSFNMNAHKWLFANQTCSPLWV KDRYSLIDALKTNPEYLEFKVSKKDTVVNYKDWQISLSRRFRFFISPFDVVFAG PISLTNLYVHAFDLYRSLKLWMVLRLYGSENLRNFIRDHVNLAKHFEDYVAQD PSFEVVTTRYFSLVCFRLAPVDGDEDQCNERNRELLAAVNSTGKIFISHTALSG KFVLRFAVGAPLTEEKHVTEAWQIIQKHASKFTRNDHY* |
| SEQ ID NO.10 | MSGTPHIAILPSPGMGHLIPMAEFAKRLVHHHNFSITFVIPTDGPPSSAYQQVLT SLPSSIDHIFLPQVDLTDVVSQSPAHPRIETLISLTVARSLSSLRTTLSSLQSSKNL VSLVVDLFGTDAFDPAIELGISPYIFFPSTAMTLSLFLYMPQLDKSVTCEFRHMT DLVRIPGCVPVRGSDLFDPVQDRTDEAYKWVIHHSNRYPMAEGVIENSFMELE HGALKYLQTVQSGKPPVYAVGPLIKMDYDVDDSGSKIIEWLDDQPVGSVLFV SFGSGGTLSYEQMTELAHGLESSQQRFLWVVRSPNQIPNSTYFSVQSQKDPLA YLPEGFLNRTEGRGLVVSNWAPQAQILSHGSTGGFMSHCGWNSILESVVHGVP |
| | IIAWPLYAEQKMNSIIVVEDVKVALRPAGVGERVVERSEITAVVKALMEGEEGK KVRNRMKELKEAAARAVSDDGASTIAIADLAQKWRSSMKH* |
| SEQ ID NO.11 | MAPVTIEKFVNQEERHLVSNRSATIPFGEYIFKRLLSIDTKSVFGVPGDFNLSLL EYLYSPSVESAGLRWVGTCNELNAAYAADGYSRYSNKIGCLITTYGVGELSAL NGIAGSFAENVKVLHIVGVAKSIDSRSSNFSDRNLHHLVPQLHDSNFKGPNHK VYHDMVKDRVACSVAYLEDIETACDQVDNVIRDIYKYSKPGYIFVPADFADMS VTCDNLVNVPRISQQDCIVYPSENQLSDIINKITSWIYSSKTPAILGDVLTDRYG VSNFLNKLICKTGIWNFSTVMGKSVIDESNPTYMGQYNGKEGLKQVYEHFEL CDLVLHFGVDINEINNGHYTFTYKPNAKIIQFHPNYIRLVDTRQGNEQMFKGIN FAPILKELYKRIDVSKLSLQYDSNVTQYTNETMRLEDPTNGQSSIITQVHLQKT MPKFLNPGDVVVCETGSFQFSVRDFAFPSQLKYISQGFFLSIGMALPAALGVGI AMQDHSNAHINGGNVKEDYKPRLILFEGDGAAQMTIQELSTILKCNIPLEVIIW NNNGYTIERAIMGPTRSYNDVMSWKWTKLFEAFGDFDGKYTNSTLIQCPSKL ALKLEELKNSNKRSGIELLEVKLGELDFPEQLKCMVEAAALKRNKK* |
| SEQ ID NO.12 | MVSEDKIEQWKATKVIGIIGLGDMGLLYANKFTDAGWGVICCDREEYYDELK EKYASAKFELVKNGHLVSRQSDYIIYSVEASNISKIVATYGPSSKVGTIVGGQTS CKLPEIEAFEKYLPKDCDIITVHSLHGPKVNTEGQPLVIINHRSQYPESFEFVNS VMACLKSKQVYLTYEEHDKITADTQAVTHAAFLSMGSAWAKIKIYPWTLGVN KWYGGLENVKVNISLRIYSNKWHVYAGLAITNPSAHQQILQYATSATELFSLM IDNKEQELTDRLLKAKQFVFGKHTGLLLLDDTILEKYSLSKSSIGNSNNCKPVP NSHLSLLAIVDSWFQLGIDPYDHMICSTPLFRIFLGVSEYLFLKPGLLEQTIDAA IHDKSFIKDDLEFVISAREWSSVVSFANFDIYKKQFQSVQKFFEPMLPEANLIG NEMIKTILSHSSDRSAAEKRNT* |
| SEQ ID NO.13 | ttaacgtcaaggagaaaaaaccccggatccatggtatcagaggataagattgagc |
| SEQ ID NO.14 | tagctagccgcggtaccaagcttactcgagttatgtatttcttttttcagcggcc |
| SEQ ID NO.15 | tccttgtaatccatcgatactagtgcggccgcctattttttatttcttttaagtgccgct |
| SEQ ID NO.16 | tcgaattcaaccctcactaaagggcggccgcatggcacctgttacaattgaaaag |
| SEQ ID NO.17 | ttgtacaactgtgctaaacagacttaaaaaagtaataattgagcgacctcatgctatac |
| SEQ ID NO.18 | Ctattggagtgatcaaaaaaaacttcaattagcgtgataccttcgagcgtcccaaaacc |
| SEQ ID NO.19 | acactcgcgagaaccaaaacaaggatcccctaaacccagt atccgctctaaccgaaaag |
| SEQ ID NO.20 | aagcgatccgtagtcctatccgggtaacactcatcgtccg cttcgagcgtcccaaaacc |
| SEQ ID NO.21 | tcgtgttcatgcagatagataacaatctatatgttgataa gagcgacctcatgctatac |
| SEQ ID NO.22 | cataaaagaccgtgtgatggcttggcatggcgatttcatt cttcgagcgtcccaaaacc |
| SEQ ID NO.23 | aaagctacatataaggaacgtgct |
| SEQ ID NO.24 | caggctgggaagcatatttgag |
| SEQ ID NO.25 | ttcatttaccggcgcactctcgcc |
| SEQ ID NO.26 | taaataagaacacccgcatgcac |
| SEQ ID NO.27 | agagcagaaagccctagtaaagc |
| SEQ ID NO.28 | aacatcgttggtaccattgggc |

## Claims

1. Use of glucosyltransferase U8GT3 and/or a poppy-derived tyrosine decarboxylase and/or a tyrosine decarboxylase mutant in improving the yield of salidroside synthesized by using yeast.

2. Use of a poppy-derived tyrosine decarboxylase and/or a tyrosine decarboxylase mutant in improving the yield of tyrosol synthesized by using yeast.

3. The use according to claim 1 or 2, wherein
the tyrosine decarboxylase mutant comprises an amino acid sequence represented by SEQ ID NO.7;
the poppy-derived tyrosine decarboxylase comprises an amino acid sequence represented by SEQ ID NO.8; and
the glucosyltransferase U8GT3 comprises an amino acid sequence represented by SEQ ID NO.10.

4. The use according to claim 1, 2 or 3, wherein
the tyrosine decarboxylase mutant comprises a nucleic acid molecule represented by SEQ ID NO.1;
the poppy-derived tyrosine decarboxylase comprises a nucleic acid molecule represented by SEQ ID NO.2; and
the glucosyltransferase U8GT3 comprises a nucleic acid molecule represented by SEQ ID NO.4.

5. A strain for producing salidroside, wherein the strain comprises a gene encoding glucosyltransferase U8GT3 and a gene encoding tyrosine decarboxylase,
preferably, the gene encoding tyrosine decarboxylase is a gene encoding a poppy-derived tyrosine decarboxylase or a gene encoding a poppy-derived tyrosine decarboxylase mutant;
more preferably,
the glucosyltransferase U8GT3 comprises an amino acid sequence represented by SEQ ID NO.10;
the poppy-derived tyrosine decarboxylase comprises an amino acid sequence represented by SEQ ID NO.8; and
the poppy-derived tyrosine decarboxylase mutant is a tyrosine decarboxylase in which the tyrosine at position 350 is mutated to phenylalanine, and comprises an amino acid sequence represented by SEQ ID NO.7,
most preferably, the yeast is *Saccharomyces cerevisiae.*

6. The strain according to claim 5, wherein
the poppy-derived tyrosine decarboxylase comprises a nucleic acid molecule represented by SEQ ID NO.2;
the tyrosine decarboxylase mutant comprises a nucleic acid molecule represented by SEQ ID NO.1; and
the glucosyltransferase U8GT3 comprises a nucleic acid molecule represented by SEQ ID NO.4.

7. The strain according to claim 5 or 6, wherein
a gene encoding phenylpyruvate decarboxylase ARO10 and/or a gene encoding benzoate dehydrogenase TYR1 is further heterologously expressed in yeast;
preferably,
the phenylpyruvate decarboxylase ARO10 comprises an amino acid sequence represented by SEQ ID NO.11; and
the benzoate dehydrogenase TYR1 comprises an amino acid sequence represented by SEQ ID NO.12;
more preferably,
the phenylpyruvate decarboxylase ARO10 comprises a nucleic acid molecule represented by SEQ ID NO.5; and
the benzoate dehydrogenase TYR1 comprises a nucleic acid molecule represented by SEQ ID NO.6.

8. A biomaterial, wherein the biomaterial is any one of the following:
A1) a nucleic acid molecule encoding a poppy-derived tyrosine decarboxylase and/or a nucleic acid molecule encoding a tyrosine decarboxylase mutant;
A2) an expression cassette comprising the nucleic acid molecule described in A1);
A3) a recombinant vector comprising the nucleic acid molecule described in A1), or a recombinant vector comprising the expression cassette described in A2);
preferably,
the tyrosine decarboxylase mutant comprises an amino acid sequence represented by SEQ ID NO.7; and
the poppy-derived tyrosine decarboxylase comprises an amino acid sequence represented by SEQ ID NO.8;
more preferably,
the tyrosine decarboxylase mutant comprises a nucleic acid molecule represented by SEQ ID NO.1; and
the poppy-derived tyrosine decarboxylase comprises the nucleic acid molecule represented by SEQ ID NO.2.

9. A biomaterial, wherein the biomaterial is any one of the following:
B1) a nucleic acid molecule encoding glucosyltransferase U8GT3;
B2) an expression cassette comprising the nucleic acid molecule described in B1);
B3) a recombinant vector comprising the nucleic acid molecule described in B1), or a recombinant vector comprising the expression cassette described in B2);
preferably,
the glucosyltransferase U8GT3 comprises an amino acid sequence represented by SEQ ID NO.10;
more preferably,
the glucosyltransferase U8GT3 comprises a nucleic acid molecule represented by SEQ ID NO.4.

10. A whole-cell catalyst, comprising the strain according to any one of claims 5 to 7 or the biomaterial according to any one of claims 8 to 9.

11. A method for producing salidroside and/or tyrosol by using yeast, wherein the salidroside and/or tyrosol are produced by fermentation using the strain according to any one of claims 5 to 8, the biomaterial according to any one of claims 9 to 10, or the whole-cell catalyst according to claim 11;
preferably,
the method comprises: adding galactose when the OD₆₀₀ is 8 to 15 during fermentation culture;
more preferably, the concentration of galactose is 1-4 g/L;
still more preferably, the fermentation time is 90-120h;
most preferably, the strain is *Saccharomyces cerevisiae.*

12. A method for producing salidroside, comprising the following steps:
synthesizing tyrosol by using a carbon source; and
synthesizing salidroside by using tyrosol;
wherein in the step of synthesizing tyrosol by using a carbon source, benzoate dehydrogenase and phenylpyruvate decarboxylase are used to synthesize tyrosol, and
in the step of synthesizing salidroside by using tyrosol, glucosyltransferase is used to synthesize salidroside,
preferably,
the glucosyltransferase is derived from *Rhodiola rosea*;
more preferably,
the glucosyltransferase comprises a nucleic acid molecule represented by SEQ ID NO.4;
the glucosyltransferase U8GT3 comprises an amino acid sequence represented by SEQ ID NO.10.

13. The method according to claim 12,
the method further comprises: synthesizing tyrosol by using poppy-derived tyrosine decarboxylase and/or tyrosine decarboxylase mutants in the step of synthesizing tyrosol by using a carbon source;
preferably,
the tyrosine decarboxylase mutant comprises an amino acid sequence represented by SEQ ID NO.7;
the poppy-derived tyrosine decarboxylase comprises an amino acid sequence represented by SEQ ID NO.8; and
the glucosyltransferase U8GT3 comprises an amino acid sequence represented by SEQ ID NO.10;
more preferably,
the tyrosine decarboxylase mutant comprises a nucleic acid molecule represented by SEQ ID NO.1;
the poppy-derived tyrosine decarboxylase comprises a nucleic acid molecule represented by SEQ ID NO.2; and
the glucosyltransferase U8GT3 comprises a nucleic acid molecule represented by SEQ ID NO.4.
